# FASCICULE DE BREVET EUROPEEN

(11) **EP 2 882 487 B1**
(45) Date de publication et mention de la délivrance du brevet: **05.06.2019**
(21) Numéro de dépôt: 13758956.0
(22) Date de dépôt: 08.08.2013
(51) Int. Cl.: A61M 39/10, A61M 39/26

(54) **CONNECTEUR A USAGE MÉDICAL**
STECKVERBINDER ZUR MEDIZINISCHEN VERWENDUNG
CONNECTOR FOR MEDICAL USE

(30) Priorité: 10.08.2012 FR 1257767
(43) Date de publication de la demande: 17.06.2015
(73) Titulaire: Cair L. G. L., 69380 Lissieu (FR)
(72) Inventeur: LOPEZ, Georges Antoine, F-69130 Ecully (FR); DELORME, Patrick, F-69630 Chaponost (FR)
(74) Mandataire: Cabinet Laurent & Charras
(86) Numéro de dépôt international: PCT/FR2013/051912
(87) Numéro de publication internationale: WO 2014/023921

(56) Documents cités:
- EP-B1- 1 890 760
- WO-A1-97/21464
- WO-A2-96/13301
- WO-A2-2011/064738

## Description

### DOMAINE TECHNIQUE

La présente invention se rapporte au secteur technique des connecteurs à usage médical, et concerne plus particulièrement un connecteur à usage médical pour la distribution d'un fluide, du type comprenant une chambre solidaire d'un raccord muni d'une aiguille, un joint élastique et une bague.

### ART ANTERIEUR

On connait du document EP 1 890 760 B1 un connecteur à usage médical selon le préambule de la revendication 1 et en particulier présentant une première extrémité dite « extrémité amont », destinée à être connectée à une tubulure dans laquelle circule un fluide ; et une seconde extrémité, désignée « extrémité aval », destinée à coopérer avec un dispositif de prélèvement ou d'injection dudit fluide par le biais d'un connecteur du type luer mâle. Dans la suite, on se réfèrera aux expressions « extrémité amont » et « extrémité aval », quel que soit le sens de circulation du fluide.

En pratique, ce connecteur présente une chambre solidaire à sa base d'un raccord constituant l'extrémité amont du connecteur proprement dit. L'extrémité libre de la chambre, à l'opposé de la base, est destinée à recevoir, par friction, l'embout d'un connecteur du type luer mâle. Le passage du fluide entre la tubulure connectée à l'extrémité amont du connecteur et l'extrémité du luer mâle est assuré par le biais d'une aiguille solidaire du corps du raccord. L'aiguille s'étend dans la chambre et débouche dans l'extrémité terminale de ladite chambre.

L'aiguille est gainée et maintenue dans l'empreinte d'un joint élastique présentant, dans l'épaisseur de son extrémité terminale libre, une fente ou équivalent permettant le passage de l'aiguille lorsque le joint élastique est compressé en position connectée du connecteur.

Ce joint élastique possède une extrémité libre tangente à celle de l'extrémité libre de la chambre et est muni d'une bague cerclant sa partie terminale jusque dans la zone en regard du ou des orifices latéraux de l'aiguille.

De plus, sur partie de la longueur de l'extrémité terminale de la chambre, la surface externe du joint élastique et/ou la surface interne ou l'épaisseur de la bague présente au moins un évidement destiné à favoriser le refoulement de la matière constitutive du joint, au moment du passage de l'aiguille.

En pratique la section interne du compartiment terminal de la chambre peut être cylindrique ou du type luer femelle, c'est-à-dire présentant un cône luer à 6%. Pour favoriser le guidage du joint élastique, la partie de la bague contenue dans le compartiment central de la chambre est de forme cylindrique.

Pour assurer l'étanchéité du système, c'est-à-dire le serrage du joint élastique entraînant la fermeture de la fente, la section externe du joint en contact avec la bague en aval de l'évidement de ladite bague, est supérieur à la section interne correspondante de la bague. En revanche, en amont du ou des évidements, la section externe du joint est sensiblement égale à la section interne de la bague.

Par ailleurs, le guidage du joint dans la chambre est assuré par la seule bague, dont tout ou partie de la paroi est en contact glissant avec la paroi correspondante de la chambre pendant toute la durée du mouvement du joint élastique, de sa position de relâchée à sa position comprimée. Du fait de la nature plastique des matériaux utilisés, il existe un minimum seulement de force de friction entre la chambre et la bague.

Cependant, le connecteur qui vient d'être décrit, qui donne une certaine satisfaction peut encore être amélioré.

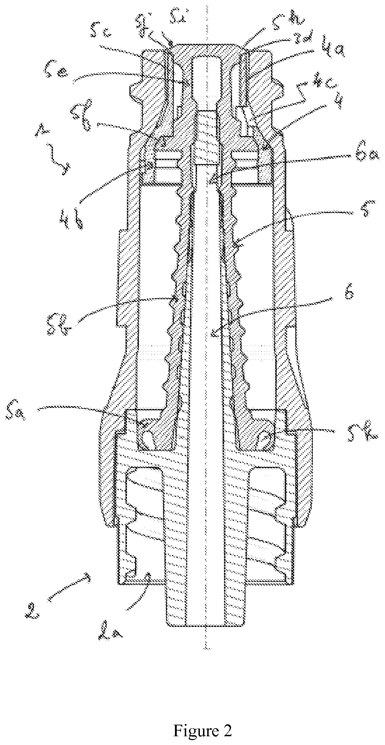

Tout d'abord, comme la bague est de forme cylindrique et que l'extrémité de la chambre a une forme luer, il subsiste nécessairement un espace entre la bague et la paroi interne de l'extrémité libre de la chambre (voir figure 2). Si petit que soit cet espace, des risques de contaminations restent possibles.

En outre, la surface de contact entre le cône luer du dispositif de prélèvement ou équivalent et le connecteur étant constitué par l'extrémité libre de la bague uniquement, l'étanchéité entre les deux éléments n'est pas optimale, les deux surfaces étant constituées d'une matière plastique rigide.

Enfin, la configuration discontinue de l'extrémité libre du connecteur, alternant du centre vers la périphérie, des matériaux indépendants et de nature différente (la partie élastique, la bague, un espace et enfin la paroi de la chambre) ne permet pas non plus de décontaminer correctement l'extrémité du connecteur après déconnexion.

Le document WO96/13331 décrit un connecteur à usage médical comprenant un raccord solidaire d'une chambre et muni en son centre d'une aiguille comprenant au moins un orifice latéral. L'aiguille est gainée, au moins dans sa partie terminale, dans l'empreinte d'un joint élastique. Le joint élastique présente une fente dans l'épaisseur de son extrémité libre, et est muni d'une bague cerclant sa partie terminale, en amont de l'orifice de l'aiguille, en position déconnectée du connecteur. Le rebord périphérique que comprend le joint élastique permet d'assurer un mouvement de coulissement et un alignement correct du joint élastique lors de l'insertion du dispositif de prélèvement ou équivalent dans la portion cylindrique du connecteur. Il est notamment précisé que ce rebord périphérique ne change pas les propriétés d'étanchéité dudit connecteur.

L'objectif est donc de mettre au point un connecteur :
- qui ne soit pas source de contamination,
- dont l'étanchéité de la connexion avec l'embout de la seringue soit maximale.

### EXPOSE DE L'INVENTION

Le but de l'invention est de remédier aux inconvénients précités en proposant un connecteur à usage médical qui permette une connexion dont l'étanchéité est optimale.

Un autre objectif de l'invention est de fournir un connecteur qui soit le moins possible sujet à la contamination bactérienne.

Pour atteindre ces objectifs, il a été mis au point un connecteur à usage médical comprenant au moins les caractéristiques de la revendication 1 avec un raccord solidaire d'une chambre. Le raccord est muni en son centre d'une aiguille s'étendant dans ladite chambre et débouchant dans son extrémité terminale. L'extrémité terminale de ladite chambre présente une section apte à recevoir, par friction, un connecteur type luer mâle. L'aiguille est munie d'au moins un orifice latéral débouchant et est comprise au moins dans sa partie terminale, incluant le ou les orifices, dans l'empreinte d'un joint élastique présentant, dans l'épaisseur de son extrémité libre, une fente ou équivalent. Le joint élastique est en outre muni d'une bague cerclant sa partie terminale au moins jusque dans la zone en regard du ou des orifices de l'aiguille, et ce lorsque le connecteur n'est pas connecté.

Selon l'invention, l'extrémité terminale du joint élastique présente un rebord périphérique déformable recouvrant l'extrémité libre de ladite bague.

Du fait du débordement du rebord périphérique déformable sur l'extrémité libre de ladite bague, l'espace présent entre ladite bague et la paroi interne de la chambre au niveau de son extrémité terminale est restreint. Cela a pour conséquence de limiter le risque de contamination bactérienne à ce niveau.

Pour limiter d'avantage encore la contamination dans l'espace formé entre la bague et la paroi de la chambre, le rebord périphérique déformable est avantageusement en contact avec la paroi interne de la chambre sur toute sa circonférence.

Parallèlement, le contact entre le cône luer mâle du dispositif de prélèvement ou équivalent et la bague n'étant plus direct mais se faisant par le biais d'un matériau élastique, l'étanchéité en position connectée se trouve renforcée.

Afin de ne laisser subsister aucun espace ou recoin susceptible de constituer des sources de contamination, entre l'extrémité terminale du joint élastique et l'extrémité libre de la chambre, le rebord périphérique déformable est en contact avec l'arrête interne de l'extrémité libre de la chambre et est avantageusement positionné à l'affleurement de celle-ci.

De préférence, dans le connecteur à usage médical selon l'invention, le rebord périphérique déformable de l'extrémité terminale du joint élastique est tangent à l'extrémité libre de la chambre. En d'autres termes, le rebord périphérique déformable et la surface de l'extrémité libre de la chambre sont dans un même plan. De cette manière, la surface terminale du connecteur est plane et une continuité de surface est assurée ente l'extrémité libre de la chambre et le joint élastique.

Dans une forme de réalisation particulière du connecteur à usage médical selon l'invention le rebord périphérique déformable de l'extrémité terminale du joint élastique présente une forme en V, dont la pointe est orientée vers la paroi interne de la chambre.

Ainsi, en position connectée, le rebord périphérique du joint est pris en sandwich entre le cône luer du dispositif de prélèvement ou équivalent et l'extrémité aval de la bague.

L'étanchéité est donc optimale. En pratique, le rebord périphérique du joint se courbe vers le haut lorsque le joint élastique passe dans une position comprimée, et subit une déformation symétrique, c'est-à-dire se courbe vers le bas, lorsque le joint élastique retourne dans sa position de relâchement.

De plus, la forme en V du rebord périphérique déformable permet de pouvoir ajuster de façon optimale l'affleurement dudit rebord par rapport à l'arrête interne de l'extrémité libre de la chambre et d'obtenir une continuité de ladite extrémité libre de la chambre avec le joint élastique. Les risques de contamination sont supprimés.

En outre, la surface libre du connecteur est continue ce qui facilite la décontamination du connecteur après utilisation.
Selon un mode de réalisation particulier, dans le connecteur selon l'invention, le joint élastique présente une forme sensiblement conique et comprend une partie formant base, une partie médiane et une partie terminale recouverte par la bague.

Avantageusement, la paroi de ladite partie médiane comprend une succession de bourrelets définissant, sur la circonférence dudit joint, une forme ondulée symétrique par rapport à un plan axial dudit joint élastique.

Selon un mode de réalisation particulier, la forme ondulée correspond à deux périodes de sinusoïde.

Dans un mode de réalisation préféré, la paroi de ladite partie médiane comprend sur la circonférence dudit joint, une succession de cavités non traversantes de formes elliptiques décalées d'un demi pas tous les 90° autour de ladite circonférence.

Ces différentes formes de réalisation de la paroi médiane du joint élastique permettent d'améliorer la souplesse dudit joint et également de favoriser le dégagement de l'aiguille. Cela permet également d'améliorer les propriétés élastiques du joint pour que celui-ci puisse adopter une position comprimée lorsque le connecteur est connecté, et revenir automatiquement dans une position de relâchement lorsque le connecteur est déconnecté.

Par ailleurs et selon une autre caractéristique, la base du joint présente une gorge périphérique continue orientée en direction amont du connecteur permettant d'éviter au volume de liquide susceptible de s'écouler dans l'espace séparant l'aiguille de la paroi interne du joint de fuir dans la chambre. En effet, sous l'effet de la pression exercée au moment de la compression du joint, le liquide vient se loger dans la gorge et exerce ainsi une pression de la base de l'élastique contre la paroi de la chambre. Tout risque de fuite arrière est ainsi évité.

### BREVE DESCRIPTION DES DESSINS

L'invention sera bien comprise et d'autres caractéristiques et avantages de l'invention ressortiront clairement de la description qui en est faite ci-après, à titre indicatif et nullement limitatif, en référence aux figures annexées, dans lesquelles :
- la figure 1 est une représentation schématique en perspective d'un connecteur à usage médical selon l'invention ;
- la figure 2 est une représentation schématique en coupe longitudinal du connecteur à usage médical selon l'invention ;
- les figures 3 à 8 sont des représentations schématiques représentant en détails l'extrémité terminale du connecteur à usage médical, ainsi que la déformation du joint élastique, à différentes étapes successives de connexion et déconnexion du connecteur selon l'invention ;
- la figure 9 est une représentation schématique en perspective d'une forme de réalisation particulière du joint élastique présent dans le connecteur à usage médical selon l'invention ;
- la figure 10 est une représentation schématique similaire à celle de la figure 9, le joint élastique étant tronqué suivant un plan passant par l'axe longitudinal dudit joint ;
- les figures 11 et 12 sont respectivement des représentations schématiques similaires à celles des figures 9 et 10, mais représentant une autre forme de réalisation du joint élastique.

### EXPOSE DETAILLE DE L'INVENTION

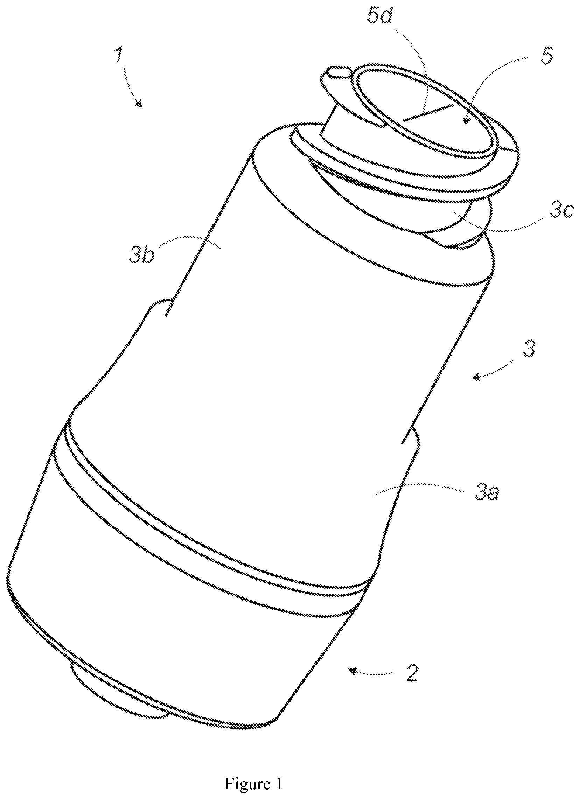

En référence à la figure 1 représentant un connecteur (1) à usage médical selon l'invention, celui-ci comprend un raccord (2) solidaire d'une chambre (3) ainsi qu'un assemblage bague (4) (non visible sur cette figure) /joint élastique (5). Le raccord (2) se présente sous la forme d'une pièce composite associant le corps du raccord (2) proprement dit et une aiguille (6) (non visible sur cette figure). La chambre (3) comprend un premier compartiment (3a), un compartiment central (3b) et un compartiment terminal (3c).

Le compartiment terminal (3c) de la chambre (3) présente une section apte à recevoir, par friction, un connecteur (7) de type luer mâle. Le premier compartiment (3a) de la chambre (3) coopère avec le raccord (2), destiné à recevoir une tubulure dans laquelle circule un fluide. En référence à la figure 2 représentant le connecteur (1) à usage médical selon l'invention, en coupe longitudinale, le corps du raccord (2) comprend un évidement (2a), muni sur sa face interne d'un pas de vis destiné à coopérer avec un pas de vis correspondant d'une tubulure ou d'un autre connecteur luer femelle dans laquelle peut circuler un fluide. Le corps du raccord (2) est muni en son centre d'une aiguille (6) s'étendant depuis le premier compartiment (3a) vers l'extrémité terminale du compartiment central (3b).

L'aiguille (6) proprement dite est munie, à proximité de son extrémité libre, elle-même obturée, de deux orifices latéraux (6a) par lesquels s'écoule le fluide. L'aiguille (6) est comprise dans l'empreinte d'un joint élastique (5) muni d'une bague (4) cerclant sa partie terminale au moins jusque dans la zone en regard des orifices (6a) de l'aiguille (6). Cette aiguille (6) présente une forme sensiblement conique de manière à favoriser son dégagement sous l'effet de la poussée exercée par le connecteur (7) type luer mâle sur l'ensemble bague (4)/joint élastique (5).

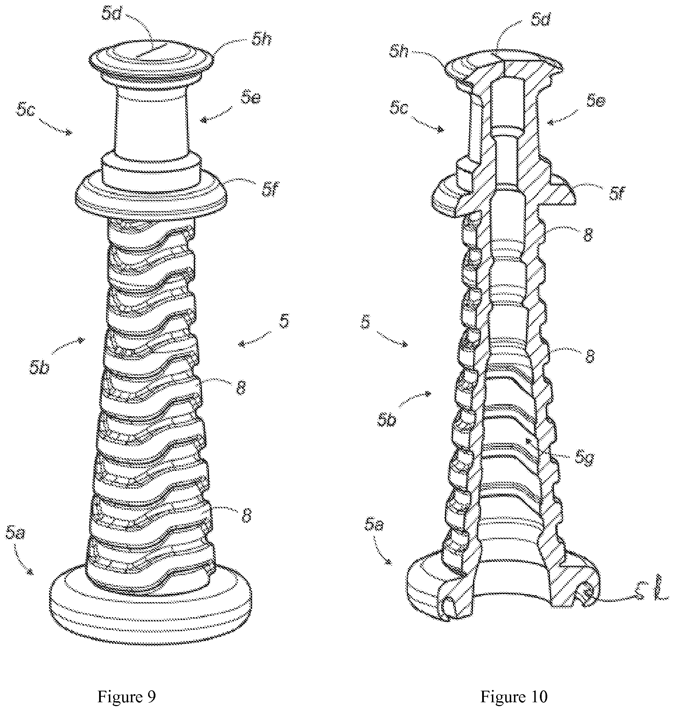

En référence aux figures 9 à 12, le joint élastique (5) est réalisé en silicone et se présente sous la forme d'un tube de section externe conique, destiné à s'étendre dans la chambre (3) depuis le corps du raccord (2) jusqu'à l'extrémité libre du compartiment terminal (3c) de la chambre (3). Le joint (5) comprend une partie formant base (5a), une partie médiane (5b) et une partie terminale (5c).

La partie formant base (5a) du joint élastique (5) est destinée à reposer sur le corps du raccord (2). En position non comprimée, c'est-à-dire en position de relâchement, ni la partie formant base (5a) du joint élastique (5), ni la partie médiane (5b) ne sont en contact avec la chambre (3). La partie formant base (5a) est munie d'une gorge continue (5k) destinée à éviter les problèmes de fuite arrière de liquide comme expliqué précédemment.

La partie médiane (5b) du joint élastique (5) présente une forme sensiblement conique et comprend dans une première forme de réalisation, illustrée aux figures 9 et 10, une succession de bourrelets (8) définissant, sur la circonférence dudit joint (5), une forme ondulée symétrique par rapport à un plan axial dudit joint élastique (5). Cette forme ondulée correspond à deux périodes de sinusoïde. En d'autres termes, les formes des bourrelets (8) peuvent être assimilées à des formes toriques affaissées sur deux côtés opposés.

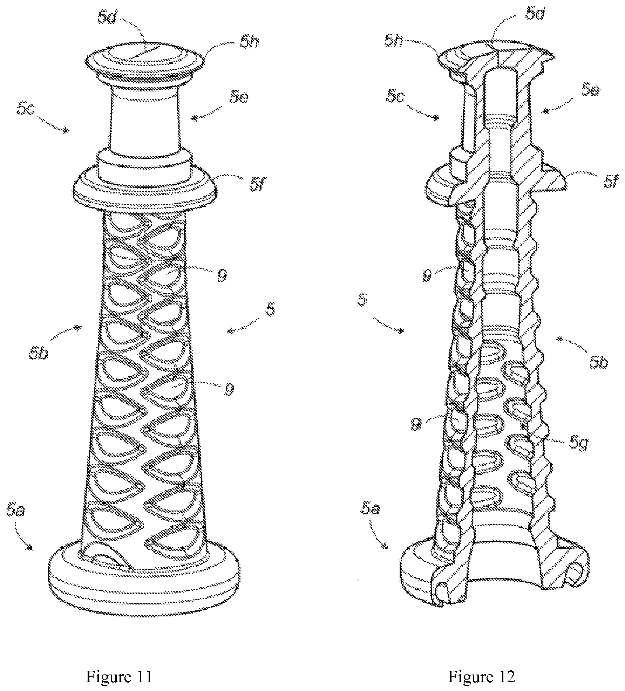

Dans une deuxième forme de réalisation, illustrée aux figures 11 et 12, la paroi de ladite partie médiane (5b) du joint élastique (5) comprend sur la circonférence dudit joint (5), une succession de cavités (9) non traversantes de formes elliptiques décalées d'un demi pas tous les 90° autour de ladite circonférence. La circonférence présente ainsi une sorte de paroi alvéolaire, dont les alvéoles sont en formes d'amandes. L'expression « décalées d'un demi pas » signifie dans le cas d'espèce, décalées d'une demi-cavité (9).

Ces différentes formes de réalisation de la paroi médiane (5b) du joint élastique (5) permettent d'améliorer la souplesse dudit joint (5) et également de favoriser le dégagement de l'aiguille (6). Cela permet également d'améliorer les propriétés élastiques du joint (5) pour que celui-ci puisse adopter une position comprimée lorsque le connecteur (1) est connecté, et revenir automatiquement dans une position de relâchement lorsque le connecteur (1) est déconnecté.

La partie terminale (5c) du joint élastique (5) est munie d'une fente (5d), permettant le passage de l'aiguille (6), lorsque le joint élastique (5) se trouve dans une position dite « comprimée », en d'autres termes, lorsque le connecteur (1) est connecté à un connecteur (7) type luer mâle. La partie terminale (5c) du joint élastique (5) comprend en outre un évidement (5e) en aval de l'extrémité de l'aiguille (6) en position non comprimée du joint élastique (5). La partie terminale (5c) présente également une collerette (5f), qui permet le guidage et le centrage de ladite partie terminale (5c) du joint élastique (5) dans le compartiment central (3b) de la chambre (3). Pour tenir compte de la forme et des dimensions du compartiment central (3b) de la chambre (3), cette collerette (5f) est également cerclée par la bague (4).

En référence aux figures 10 et 12, le joint élastique (5) présente en son centre, une empreinte (5g) de forme correspondante à celle de l'aiguille (6), l'empreinte (5g) étant toutefois légèrement plus longue que l'aiguille (6).

La section interne du joint élastique (5) est inférieure à la section de l'aiguille (6) y compris dans la zone terminale de l'aiguille (6) recouverte par la bague (4) et incluant les orifices latéraux (6a), au niveau de laquelle la section interne du joint élastique (5) est inférieure à la section de l'aiguille (6).

La bague (4) est positionnée à l'extrémité du joint élastique (5) et présente deux tronçons de sections différentes, respectivement un premier tronçon cylindrique (4a) cerclant la partie terminale (5c) du joint élastique (5) sur une longueur correspondant à la longueur du compartiment terminal (3c) et de section sensiblement égale à la section dudit compartiment terminal (3c) et un second tronçon cylindrique (4b), de section supérieure sensiblement égale à la section correspondante du compartiment central (3b) et recouvrant la collerette (5f) du joint élastique (5). La bague (4) est réalisée en un matériau rigide ou semi-rigide, éventuellement en bi-matière avec le joint élastique (5), ou séparément, bague (4) et joint (5) étant rendus solidaires l'un de l'autre notamment par collage ou simple apposition. La section externe du joint (5) est sensiblement égale à la section interne de la bague (4) sur leur zone de recouvrement.

Selon une caractéristique de l'invention, l'extrémité terminale du joint élastique (5) présente un rebord périphérique déformable (5h) débordant sur l'extrémité de ladite bague (4). Ce rebord périphérique (5h) présente un chanfrein périphérique supérieur (5i) et un chanfrein périphérique inférieur (5j). Les deux chanfreins (5i, 5j) sont réalisés de sortes qu'ils se rejoignent pour donner une forme en V audit rebord périphérique (5h), dont la pointe est orientée vers la paroi interne de la chambre (3).

La forme en V du rebord périphérique déformable (5h) permet de pouvoir ajuster de façon optimale l'affleurement dudit rebord (5h) par rapport à l'arrête interne (3d) de l'extrémité libre de la chambre (3) et d'obtenir une continuité de ladite extrémité libre de la chambre (3) avec le joint élastique (5). Les risques de contamination par les bactéries sont très nettement supprimés

En position de « relâchement » du joint élastique (5), et afin de ne laisser subsister aucun espace ou recoin, susceptible de devenir des sources de contamination, entre l'extrémité terminale du joint élastique (5) et l'extrémité libre de la chambre (3), la pointe du V du rebord périphérique déformable (5h) est en contact avec l'arrête interne (3d) de l'extrémité libre de la chambre (3) et est à l'affleurement de celle-ci.

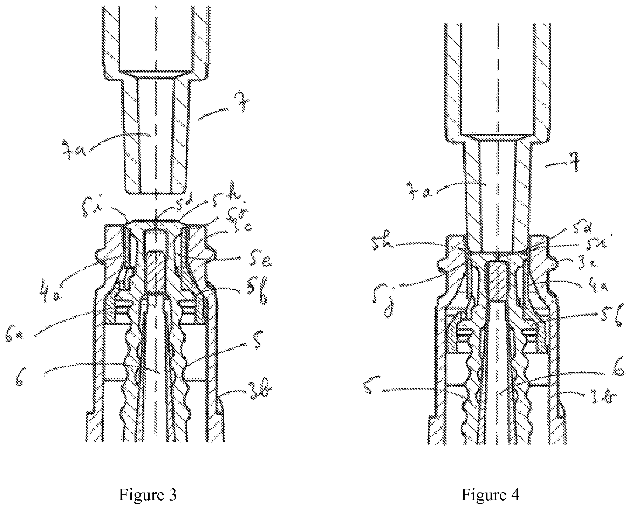

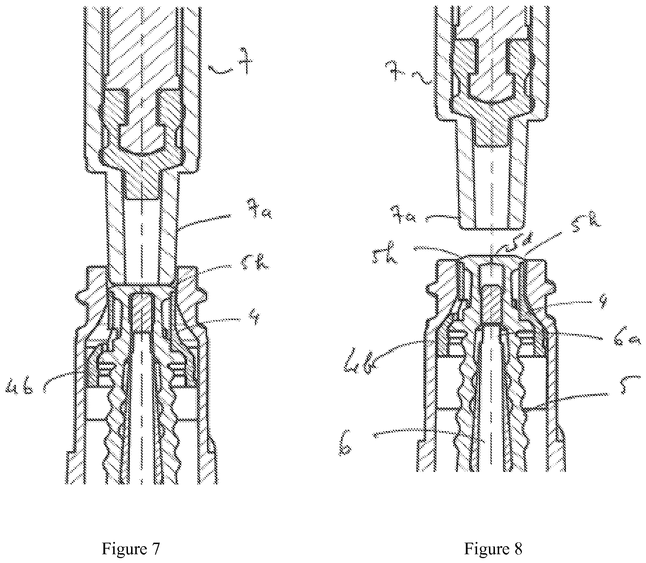

En référence aux figures 3 à 8, la paroi interne du compartiment terminal (3c) de la chambre (3) présentant un profil luer, c'est-à-dire en cône à 6%, le rebord périphérique déformable (5h) est destiné à se déformer lorsque le joint passe dans une position comprimée, ou revient dans une position de relâchement. Ainsi, lorsque le joint élastique (5) passe dans une position comprimée, la déformation du rebord périphérique déformable (5h) contre la paroi interne luer de l'extrémité libre de la chambre (3), est facilitée. Plus précisément, ledit rebord périphérique (5h) pris en sandwich entre le cône (7a) du connecteur (7) et la bague (4) se courbe vers le haut lorsque le joint élastique (5) passe dans une position comprimée, et subit une déformation symétrique, c'est-à-dire se courbe vers le bas lorsque le joint élastique (5) retourne dans sa position de relâchement
Comme le montre la figure 2, la partie terminale et plus particulièrement le premier tronçon cylindrique (4a) de la bague (4) présente deux ajourements (4c) destinés à favoriser le refoulement de la matière plastique au moment du passage de l'aiguille (6) sous l'effet de la poussée créée par la mise en place du connecteur (7) type luer mâle.

Comme illustré sur la figure 2, le compartiment central (3b) et le compartiment terminal (3c) sont reliés par un épaulement servant de butée à la bague (4) cerclant l'extrémité terminale (5c) du joint élastique (5) en position de repos, c'est-à-dire en position de relâchement du joint, garantissant ainsi que le rebord périphérique déformable (5h) du joint élastique (5) soit à l'affleurement de l'arrête interne (3d) de l'extrémité libre de la chambre (3).

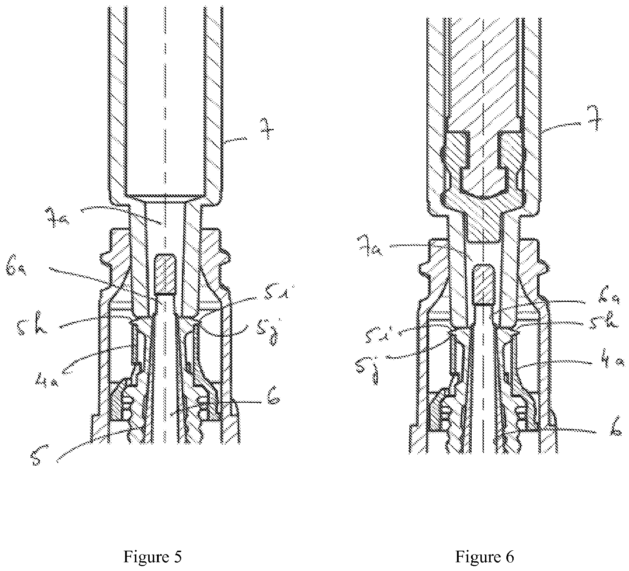

La position connectée est plus précisément représentée sur la figure 6 par la mise en place d'un connecteur (7) type luer mâle. Ce connecteur (7) est en outre muni d'un cône luer (7a), destiné à être insérée dans la lumière du compartiment terminal (3c) de la chambre (3). En pratique, le cône (7a) vient prendre appui par son extrémité sur l'extrémité libre du joint élastique (5). Cet appui provoque le déplacement du joint élastique (5) le long du fût de l'aiguille (6) en direction du raccord (2), puis le passage de la pointe de l'aiguille (6) au travers de la fente (5d), facilité par les évidements (5e), et enfin la libération des orifices latéraux (6a) et autorisant le passage du fluide. La présence du rebord périphérique (5h) permet, lors de l'appui du cône (7a) du connecteur (7) luer mâle sur le joint élastique (5), d'écraser ledit rebord périphérique (5h) entre ledit cône (7a) et la bague (4). Il en résulte un meilleur transfert d'effort et un meilleur centrage de l'ensemble bague (4)/joint (5) par rapport à la chambre (3). La connexion est réalisée de manière optimale. En outre l'étanchéité du système est ainsi garantie

En position connectée, la partie libre de l'aiguille (6) est donc dans sa totalité, contenue dans le canal intérieur du cône (7a), permettant ainsi la transmission du fluide de connecteur (1) à connecteur (7). Le mouvement de la bague (4) pendant cette opération, est un mouvement axial homogène et uniforme de par le contact permanent des parois de la section de la bague (4) avec le compartiment central (3b) de la chambre (3) tout au long du mouvement, moyennant un minimum de friction. Comme le montre la figure 6, en position comprimée du joint élastique (5), la partie de la bague (4) de faible section est en contact ni avec le compartiment terminal (3c) de la chambre (3), ni avec le compartiment central (3b) de la chambre (3). En pratique, le volume du compartiment central (3b) de la chambre (3) est prévu pour pouvoir contenir le volume du joint (5) en position comprimée.

Comme il ressort de ce qui précède, l'invention fournit un connecteur (1) à usage médical donnant entière satisfaction d'utilisation. On note en particulier l'étanchéité totale du système obtenue en amont de la fente (5d) terminale du joint élastique (5). Le connecteur (1) selon l'invention permet en outre une étanchéité maximale de la connexion avec un autre connecteur (7) luer mâle, une diminution accrue des risques de contaminations bactérienne de l'extrémité terminale de la chambre (3), et un centrage optimal du joint élastique (5) par rapport à l'extrémité terminale de la chambre (3).

## Revendications

1. Connecteur (1) à usage médical comprenant un raccord (2) solidaire d'une chambre (3), le raccord (2) étant muni en son centre d'une aiguille (6) s'étendant dans ladite chambre (3) et débouchant dans son extrémité terminale, ladite extrémité terminale présentant une section apte à recevoir, par friction, un connecteur type luer mâle (7), l'aiguille (6) étant munie d'au moins un orifice latéral (6a) débouchant et étant comprise au moins dans sa partie terminale, incluant le ou les orifices (6a), dans l'empreinte (5g) d'un joint élastique (5) présentant, dans l'épaisseur de son extrémité libre, une fente (5d) ou équivalent, le joint élastique (5) étant en outre muni d'une bague (4) cerclant sa partie terminale (5c) au moins jusque dans la zone en regard du ou des orifices (6a) de l'aiguille (6) en position déconnectée du connecteur, ***caractérisé en ce que*** l'extrémité terminale (5c) du joint élastique (5) présente un rebord périphérique déformable (5h) recouvrant et débordant l'extrémité libre de ladite bague (4).

2. Connecteur (1) selon la revendication 1, ***caractérisé en ce que*** le rebord périphérique déformable (5h) est en contact avec la paroi interne de la chambre (3) sur toute sa circonférence hors connexion.

3. Connecteur (1) selon l'une des revendications précédentes, ***caractérisé en ce que*** le rebord périphérique déformable (5h) est en contact avec l'arrête interne (3d) de l'extrémité libre de la chambre (3).

4. Connecteur (1) selon l'une des revendications précédentes, ***caractérisé en ce que*** le rebord périphérique déformable (5h) est positionné à l'affleurement de l'arrête interne (3d) de l'extrémité libre de la chambre (3).

5. Connecteur (1) selon l'une des revendications précédentes, ***caractérisé en ce que*** le rebord périphérique déformable (5h) est tangent à l'extrémité libre de la chambre (3).

6. Connecteur (1) selon l'une des revendications précédentes, ***caractérisé en ce que*** le rebord périphérique déformable (5h) présente une forme en V dont la pointe est orientée vers la paroi interne de la chambre (3).

7. Connecteur (1) selon l'une des revendications précédentes, ***caractérisé en ce que*** le joint élastique (5) présente une forme sensiblement conique et comprend une partie formant base (5a), une partie médiane (5b) et une partie terminale (5c) recouverte par la bague (4).

8. Connecteur (1) selon la revendication 7, ***caractérisé en ce que*** la paroi de ladite partie médiane (5b) comprend une succession de bourrelets (8) définissant, sur la circonférence dudit joint (5), une forme ondulée symétrique par rapport à un plan axial dudit joint élastique (5).

9. Connecteur (1) selon la revendication 8, ***caractérisé en ce que*** la forme ondulée correspond à deux périodes de sinusoïde.

10. Connecteur (1) selon la revendication 7, ***caractérisé en ce que*** la paroi de ladite partie médiane (5b) comprend sur la circonférence dudit joint (5), une succession de cavités (9) non traversantes de formes elliptiques décalées d'un demi pas tous les 90° autour de ladite circonférence.

11. Connecteur (1) selon l'une des revendications précédentes, ***caractérisé en ce que*** la base du joint élastique est muni d'une gorge périphérique (5k) continue orientée en direction amont du connecteur.

## Patentansprüche

1. Steckverbinder (1) für medizinische Zwecke, mit einer Kupplung (2), die mit einer Kammer (3) fest verbunden ist und in ihrem Zentrum mit einer Nadel (6) versehen ist, die in diese Kammer (3) hineinreicht und in ihrem Endabschnitt mündet, wobei dieser Endabschnitt einen Abschnitt umfasst, in dem ein Luer-Stecker (7) reibschlüssig untergebracht werden kann, die Nadel (6) ist dabei mit mindestens einer seitlichen Öffnung (6a) versehen, die zumindest mit ihrem Endstück, einschließlich der Öffnung(en) (6a), im Hohlraum (5g) einer elastischen Dichtung (5) mündet und sich darin befindet, diese Dichtung weist in der Dicke ihres freien Endstücks einen Schlitz (5d) oder ähnliches auf, die elastische Dichtung (5) ist außerdem mit einem Ring (4) versehen, der ihr Abschlussende (5c) mindestens in der Zone gegenüber der oder den Öffnung(en) (6a) der Nadel (6) in der ausgesteckten Position des Steckverbinders umgibt, ***dadurch gekennzeichnet, dass*** das Abschlussende (5c) der elastischen Dichtung (5) einen verformbaren Umfangsflansch (5h) aufweist, der das freie Endstück des Rings (4) bedeckt und überragt.

2. Steckverbinder (1) gemäß Anspruch 1 ***dadurch gekennzeichnet, dass*** der verformbare Umfangsflansch (5h) über den gesamten Umfang außer der Verbindung Kontakt zur Innenwand der Kammer (3) hat.

3. Steckverbinder (1) gemäß einem der vorangehenden Ansprüche, ***dadurch gekennzeichnet, dass*** der verformbare Umfangsflansch (5h) Kontakt mit der Innenkante (3d) des freien Endes der Kammer (3) hat.

4. Steckverbinder (1) gemäß einem der vorangehenden Ansprüche, ***dadurch gekennzeichnet, dass*** der verformbare Umfangsflansch (5h) anschließend an die Innenkante (3d) des freien Endes der Kammer (3) angeordnet ist.

5. Steckverbinder (1) gemäß einem der vorangehenden Ansprüche, ***dadurch gekennzeichnet, dass*** der verformbare Umfangsflansch (5h) tangential zum freien Ende der Kammer (3) ist.

6. Steckverbinder (1) gemäß einem der vorangehenden Ansprüche, ***dadurch gekennzeichnet, dass*** der verformbare Umfangsflansch (5h) eine V- Form hat, deren Spitze zur Innenwand der Kammer (3) hin orientiert ist.

7. Steckverbinder (1) gemäß einem der vorangehenden Ansprüche, ***dadurch gekennzeichnet, dass*** die elastische Dichtung (5) eine im Wesentlichen konische Form hat und einen Teil umfasst, der eine Basis (5a), einen Mittelteil (5b) und einen Abschlussteil (5c), der vom Ring (4) bedeckt wird, bildet.

8. Steckverbinder (1) gemäß Anspruch 7, ***dadurch gekennzeichnet, dass*** die Wand dieses Mittelteils (5b) aufeinanderfolgende Wülste (8) umfasst, die auf dem Umfang dieser Dichtung (5) eine Wellenform, symmetrisch, bezogen auf eine axiale Ebene dieser elastischen Dichtung (5), definieren.

9. Steckverbinder (1) gemäß Anspruch 8, ***dadurch gekennzeichnet, dass*** die Wellenform zwei Sinuskurvenperioden entspricht.

10. Steckverbinder (1) gemäß Anspruch 7, ***dadurch gekennzeichnet, dass*** die Wand dieses Mittelteils (5b) auf dem Umfang dieser Dichtung (5) aufeinanderfolgende, nicht hindurchführende Vertiefungen (9) enthält, mit elliptischer Form, um einen Halbschritt alle 90° um diesen Umfang versetzt.

11. Steckverbinder (1) gemäß einem der vorangehenden Ansprüche, ***dadurch gekennzeichnet, dass*** die Basis der elastische Dichtung mit einer durchgehend umlaufenden Kehle (5k) versehen ist, die in Richtung auf den Steckverbinder hin ausgerichtet ist.

## Claims

1. A connector (1) for a medical use comprising a fitting (2) secured to a chamber (3), the fitting (2) being provided at its center with a needle (6) extending in said chamber (3) and emerging into its terminal end, said terminal end having a cross-section capable of receiving, by friction, a connector of male Luer type (7), the needle (6) being provided with at least one lateral through hole (6a) and being placed at least in its terminal portion, including the hole(s) (6a), in the recess (5g) of an elastic seal (5) having, across the thickness of its free end, a slit (5d) or the like, the elastic seal (5) being further provided with a ring (4) encircling its terminal portion (5c) at least all the way to the area opposite the hole(s) (6a) of the needle (6), the connector (1) in its disconnected position, ***characterized in that*** the terminal end (5c) of the elastic seal (5) has a deformable peripheral rim (5h) covering and running over the free end of said ring (4).

2. The connector (1) of claim 1, ***characterized in that*** the deformable peripheral rim (5h) is in contact with the internal wall of the chamber (3) along its entire circumference, the connector (1) being disconnected.

3. The connector (1) of any of the foregoing claims, ***characterized in that*** the deformable peripheral rim (5h) is in contact with the internal edge (3d) of the free end of the chamber (3).

4. The connector (1) of any of the foregoing claims, ***characterized in that*** the deformable peripheral rim (5h) is positioned flush with the internal edge (3d) of the free end of the chamber (3).

5. The connector (1) of any of the foregoing claims, ***characterized in that*** the deformable peripheral rim (5h) is tangent to the free end of the chamber (3).

6. The connector (1) of any of the foregoing claims, ***characterized in that*** the deformable peripheral rim (5h) has the shape of a V directed towards the internal wall of the chamber (3).

7. The connector (1) of any of the foregoing claims, ***characterized in that*** the elastic seal (5) has a substantially conical shape and comprises a base-forming portion (5a), a median portion (5b), and a terminal portion (5c) covered with the ring (4).

8. The connector (1) of claim 7, ***characterized in that*** the wall of said median portion (5b) comprises a succession of beads (8) defining, along the circumference of said seal (5), a wavy shape symmetrical with respect to an axial plane of said elastic seal (5).

9. The connector (1) of claim 8, ***characterized in that*** the wavy shape corresponds to two sinusoid periods.

10. The connector (1) of claim 7, ***characterized in that*** the wall of said median portion (5b) comprises along the circumference of said seal (5), a succession of non-through cavities (9) of elliptic shapes offset by one half-step every 90° around said circumference.

11. The connector (1) of any of the foregoing claims, ***characterized in that*** the base of the elastic seal is provided with a continuous peripheral groove (5k) directed upstream of the connector.
